# EUROPEAN PATENT SPECIFICATION

(11) **EP 1 242 062 B1**
(45) Date of publication and mention of the grant of the patent: **28.07.2004**
(21) Application number: 00991243.7
(22) Date of filing: 21.12.2000
(51) Int. Cl.: A61K 31/18, A61K 31/192

(54) **ANHYDROUS GEL COMPRISING NSAID FOR TOPICAL ADMINISTRATION TO THE ORAL CAVITY**
NSAID ENTHALTENDES WASSERFREIES GEL ZUR TOPISCHEN ANWENDUNG IN DER MUNDHÖHLE
COMPOSITIONS PHARMACEUTIQUES POUR L'ADMINISTRATION TOPIQUE DANS LA CAVITE BUCCALE DE PRODUITS ANTI-INFLAMMATOIRES NON STEROIDES

(30) Priority: 29.12.1999 IT MI992736
(43) Date of publication of application: 25.09.2002
(73) Proprietor: Cosmo S.p.A., 20020 Lainate Milano (IT)
(72) Inventor: PEDRANI, Massimo, London W1R 9FB (GB); AJANI, Mauro, London W1R 9FB (GB); VILLA, Roberto, 20020 lainate (MI) (IT)
(74) Representative: Pistolesi, Roberto
(86) International application number: PCT/EP2000/013080
(87) International publication number: WO 2001/049276

(56) References cited:
- EP-A- 0 367 382
- WO-A-93/10791
- US-A- 4 263 313
- US-A- 4 883 660
- US-A- 5 314 915
- RAHMAN M.S. ET AL: "Medicament release from ointment bases: V. Naproxen in-vitro release and in-vivo percutaneous absorption in rabbits." DRUG DEVELOPMENT AND INDUSTRIAL PHARMACY, (1990) 16/4 (651-672). , XP002169880

## Description

### Disclosure of the invention.

The present invention relates to pharmaceutical compositions for the topical administration in the oral cavity of non-steroid anti-inflammatory drugs useful for the stomatologic, mouth and oral cavity anti-inflammatory and analgesic therapies.

The use of non-steroid anti-inflammatory drugs is the first therapeutical intervention in case of inflammatory diseases of the mouth and dental system. This class of medicaments is, in fact, known to have some analgesic activity which is only exerted when blood drug levels reach a comparatively higher concentration than that necessary to exert the real anti-inflammatory activity. To obtain a high drug concentration in blood, the so-called "fast release" formulations are made use of, wherein the dissolution of the active ingredient is promoted by suitably formulating it, for example through salification with high solubility counterions or amorphous dispersion of the drug on high solubility carriers: the acceleration of the drug dissolution rate immediately gives rise to a concentration peak at the absorption site, which corresponds to a higher concentration peak in the circulation thus exceeding the analgesic activity concentration threshold.

Therefore, in the case of systemic administration, a drug high concentration in blood is mandatory to obtain a suitable concentration reaching the analgesic threshold also at the oral district. As a consequence, high amounts of active ingredient are administered which thus exerts its action not only at the desired peripheral district, but at the whole body, even if it is not necessary.

Such drawback could be overcome by topically administering the active ingredient formulated in pharmaceutical compositions providing sufficient absorption to reach effective concentration levels. At present, the commercially available formulations useful for the topical administration of anti-inflammatory drugs do not meet such requirement: they usually are, in fact, solutions or collutories in which the drug is dissolved in a mainly aqueous carrier, which remains in contact with the absorption site for a short time due to the immediate dilution with saliva and is quickly removed from the administration site, thus not reaching effective absorption levels. Formulation in gel are also known, US 4 883 660 discloses anhydrous gels comprising anti-inflammatory drugs for the topical administration to the oral cavity; US 5 314 915 discloses a bioahesive pharmaceutical gel comprising anti-inflammatory drugs for oral use.
It has now surprisingly been found that the administration of a non-steroid anti-inflammatory drug in a carrier with suitable anhydricity and amphiphilia characteristics promotes the absorption of the drug at the administration topical site until attaining an analgesically effective concentration, without making use of the higher dosages which would be necessary to obtain a comparable activity through systemic administration.

The invention therefore relates to compositions for the topical administration in the oral cavity of non-steroid anti-inflammatory drugs in the form of anhydrous semisolid gelled formulations.

Poorly hydrophilic active ingredients, which are difficult to formulate in the conventional aqueous carriers, are most suitable for this formulation in that they are hardly washed and diluted in biological fluids; examples of said drugs include Nimesulide, which is sparingly soluble in aqueous medium, Diclofenac acid and the arylpropionic anti-inflammatory derivatives, in a carrier with acidity characteristics which limit ionic dissociation.

The compositions of the invention contain an anhydrous solvent combined with viscosity-increasing/bioadhesive agents.

The suitable solvents for use in the invention are selected from glycerol, propylene glycol, polyethylene glycol 200 or 400 or diethylene glycol monoethyl ether. Glycerol, polyethylene glycol 200 or 400 and diethylene glycol monoethyl ether (Transcutol®) are particularly preferred.

The viscosity-increasing/bioadhesive agent is a carboxyvinyl polymer. The compositions of the invention can further contain compounds providing the carrier with specific organoleptic characteristics, such as flavours, flavour and pH adjusters, dyes, sweetening agents, surfactants.

The compositions of the invention in the form of anhydrous gel will preferably contain the active ingredient in a range from 0.5 to 10%, preferably from 1 to 5%.

The compositions of the invention will typically have viscosity ranging from 1,000 to 200,000 mPa^{·}s (cps), preferably 5,000-100,000 mPa^{·}s (cps).

The percentages of anhydrous solvent in the compositions can range from 5 to 50% by weight in the case of polyethylene glycol 200 or 400, from 5 to 30% in the case of glycerol and from 10 to 80% in the case of Transcutol®).

On the other hand, the bioadhesive agents will usually be present in 0.1 to 5% weight percentages.

The invention further relates to the process for the preparation of the formulations, which comprises the dissolution of the active ingredient in the anhydrous solvent and the successive addition of the viscosity-increasing/bioadhesive agents and optionally other excipients.

The formulations of the invention will usually be presented in the form of a anhydrous gel, clear, easy to spread by means of a spatula or more simply with a finger on the mucosae under treatment. The anhydricity of the preparation allows for the active ingredient to remain for a long time at the administration site, which is also favored by the hydration-induced bioadhesion which secures grafting through the polymer hydrophilic chains of the viscosity-increasing bioadhesive, thus ensuring a long-lasting, effective absorption of the active ingredient.

The following examples further illustrate the characteristics of the invention.

### Example 1

30 g of Nimesulide are dissolved in a mixture prepared with 400 g Polyethylene glycol 200, 350 g of Polyethylene glycol 400, 100 g of propylene glycol and 100 g of diethylene glycol monoethyl ether. After dissolution of the active ingredient, 20 g of carboxyvinyl polymer known as Carbomer 934 ® are added: the final product is an anhydrous gel, smooth and easy to spread, which is distributed in aluminium tubes internally coated with polyethene.

### Example 2

300 g of Nimesulide are dissolved in a mixture of anhydrous solvents consisting of 4 kg of diethylene glycol monoethyl ether, 3400 g of Polyethylene glycol 400, 1 kg of propylene glycol. The resulting mixture is added with 1 kg of glycerol and 300 g of Carbomer ® viscosity agent. The formulation is flavored with small amounts of flavor before being distributed in aluminium tubes internally coated with polyethene.

This formulation was clinically tested to reveal an absorption profile of 500 to 200,000 mPa^{·}s (cPs).

### Example 3

20 g of Ketoprofen are dissolved in a mixture consisting of 300 g of polyethylene glycol 400, 360 g of diethylene glycol monoethyl ether, 150 g of glycerol, 150 g of propylene glycol. The resulting solution is thickened by addition of 20 g of carboxyvinyl polymer and partitioned in aluminium tubes internally coated with polyethene. The final formulation is a clear anhydrous gel which is easy to spread and shows no tolerability problems.

## Claims

1. Compositions for the topical administration in the oral cavity of non-steroid anti-inflammatory drugs in the form of anhydrous gelled semisolid formulations wherein the active ingredient is dissolved in an anhydrous solvent selected from glycerol, propylene glycol, polyethylene glycol 200 or 400, diethylene glycol monoethyl ether and containing a viscosity-increasing/bioadhesive carboxyvinyl polymer.

2. Compositions as claimed in claim 1 wherein the non-steroid anti-inflammatory drugs selected from Nimesulide, Ketoprofen, Diclofenac and/or arylpropionic compounds.

3. Compositions as claimed in the above claims containing Nimesulide.

4. Compositions as claimed in claim 3, wherein Nimesulide or Ketoprofen are present in concentrations ranging from 0.5 to 10% w/w.

5. Compositions as claimed in claim 4, wherein Nimesulide or Ketoprofen are present in concentrations ranging from 1 to 5% w/w.

6. Compositions as claimed in any one of the above claims further containing one or more excipients selected from surfactants, flavours, flavour or pH adjusters, sweetening agents, dyes.

7. Compositions as claimed in any one of the above claims with viscosities ranging from 1,000 to 200,000 mPa^{·}s (cPs).

8. A process for the preparation of the formulations of claims 1-7 which comprises the dissolution of the active ingredient in the anhydrous solvent and the successive addition of the viscosity-increasing/bioadhesive agent and optionally other excipients.

## Patentansprüche

1. Zusammensetzungen zur topischen Verabreichung von nicht-steroidalen entzündungshemmenden Arzneistoffen in Form von wasserfreien gelierten halbfesten Formulierungen in der Mundhöhle, in denen der Wirkstoff in einem wasserfreien Lösungsmittel gelöst ist, das aus Glycerin, Propylenglycol, Polyethylenglycol 200 oder 400, Diethylenglycolmonoethylether ausgewählt ist, und die ein viskositätserhöhendes/bioadhäsives Carboxyvinylpolymer enthalten.

2. Zusammensetzungen nach Anspruch 1, in denen der nicht-steroidale entzündungshemmende Arzneistoff aus Nimesulid, Ketoprofen, Diclofenac und/oder Arylpropionsäure-Verbindungen ausgewählt ist.

3. Zusammensetzungen nach den obigen Ansprüchen, die Nimesulid enthalten.

4. Zusammensetzungen nach Anspruch 3, in denen Nimesulid oder Ketoprofen in Konzentrationen im Bereich von 0,5 bis 10 % Gew./Gew. vorliegt.

5. Zusammensetzungen nach Anspruch 4, in denen Nimesulid oder Ketoprofen in Konzentrationen im Bereich von 1 bis 5 % Gew./Gew. vorliegt.

6. Zusammensetzungen nach irgendeinem der obigen Ansprüche, die weiter einen oder mehrere Hilfsstoffe enthalten, die aus Tensiden, Geschmacksstoffen, Geschmacks- oder pH-Reglern, Süßungsmitteln, Farbstoffen ausgewählt sind.

7. Zusammensetzungen nach irgendeinem der obigen Ansprüche mit Viskositäten im Bereich von 1.000 bis 200.000 mPa·s (cPs).

8. Verfahren zur Herstellung der Formulierungen der Ansprüche 1 - 7, welches die Auflösung des Wirkstoffs in dem wasserfreien Lösungsmittel und die folgende Zugabe des viskositätserhöhenden/ bioadhäsiven Mittels und gegebenenfalls anderer Hilfsstoffe umfasst.

## Revendications

1. Compositions pour l'administration topique dans la cavité buccale de médicaments anti-inflammatoires non-stéroïdiens sous la forme de formulations semi-solides gélifiées anhydres, dans lesquelles le principe actif est dissout dans un solvant anhydre choisi parmi le glycérol, le propylène glycol, le polyéthylène glycol 200 ou 400, l'éther monoéthylique du diéthylène glycol et contenant un polymère de carboxyvinyle bio-adhésif augmentant la viscosité.

2. Compositions selon la revendication 1, dans lesquelles les médicaments anti-inflammatoires non stéroïdiens sont choisis parmi le Nimésulide, le Kétoprofene, le Diclofénac et/ou des composés arylproprioniques.

3. Compositions selon les revendications précédentes contenant du Nimésulide.

4. Compositions selon la revendication 3, dans lesquelles du Nimésulide ou du Kétoprofène est présent dans des concentrations situées dans la plage de 0,5 à 10 % p/p.

5. Compositions selon la revendication 4, dans lesquelles du Nimésulide ou du Kétoprofène est présent dans des concentrations situées dans la plage de 1 à 5 % p/p.

6. Compositions selon l'une quelconque des revendications précédentes contenant en outre un ou plusieurs excipients choisis parmi des surfactants, des parfums, des agents ajustant le parfum ou le pH, des agents édulcorants, des colorants.

7. Compositions selon l'une quelconque des revendications précédentes avec des viscosités situées dans la plage de 1000 à 200 000 mPa^{·}s (cPs).

8. Procédé de préparation des formulations des revendications 1 à 7 qui comprend la dissolution du principe actif dans le solvant anhydre et l'addition successive de l'agent bio-adhésif augmentant la viscosité et éventuellement d'autres excipients.
